(19) Europäisches Patentamt
European Patent Office
Office européen des brevets

(11) **EP 3 974 795 A1**

(12) **EUROPEAN PATENT APPLICATION**
published in accordance with Art. 153(4) EPC

(43) Date of publication:
**30.03.2022 Bulletin 2022/13**

(21) Application number: **20808671.0**

(22) Date of filing: **21.05.2020**

(51) International Patent Classification (IPC):
**G01L 1/20** (2006.01)    **A61M 25/00** (2006.01)

(52) Cooperative Patent Classification (CPC):
**A61M 25/00; G01L 1/20**

(86) International application number:
**PCT/CN2020/091573**

(87) International publication number:
**WO 2020/233672 (26.11.2020 Gazette 2020/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.05.2019 CN 201910422539**

(71) Applicant: **Shanghai Zuoxin Medical Technology Co., LTD.**
**Shanghai 200000 (CN)**

(72) Inventors:
• **ZHOU, Yi**
  **Shanghai 200000 (CN)**
• **ZHOU, Ting**
  **Shanghai 200000 (CN)**
• **YAO, Yao**
  **Shanghai 200000 (CN)**
• **LI, Junfei**
  **Shanghai 200000 (CN)**
• **WANG, Ben**
  **Shanghai 200000 (CN)**

(74) Representative: **Arnold & Siedsma**
**Bezuidenhoutseweg 57**
**2594 AC The Hague (NL)**

(54) **MEDICAL DEVICE AND MEDICAL CATHETER THEREOF**

(57) This application relates to a medical device and a medical catheter thereof. The medical catheter includes a catheter body and a resistance strain material layer, wherein the resistance strain material layer is attached to the surface of the catheter body, or buried in the catheter body. The resistance strain material layer is deformable with the bending and/or twisting of the catheter body.

FIG. 1

## Description

## TECHNICAL FIELD

The present application relates to the technical field of medical catheters, in particular to a medical device and a medical catheter thereof.

## BACKGROUND

In the process of interventional surgery, medical catheters (for example, delivery sheath catheters) are usually used for related operations. For example, the delivery sheath catheter is used to establish a delivery channel, and an interventional device is pushed to the diseased location along the delivery sheath catheter. The delivery sheath catheter is required to enter a body to build the delivery channel, thus the force must be managed to avoid injury when the delivery sheath catheter is put into the body.

However, there are still some problems in measuring the force on and deformation of the delivery sheath catheter. For example, Chinese patent application No. CN201520229910.7 discloses a ureteral introduction sheath with real-time pressure measurement and adsorption capabilities, wherein a pressure-sensing channel is established on a side of the introduction sheath and is connected with a pressure-sensing connector. The pressure-sensing connector is connected to an external pressure sensor so that the pressure at the end of the introduction sheath can be measured. However, it is not conducive to the placement of other functional catheters inside the delivery sheath catheter, since the pressure-sensing channel is installed on the side of the introduction sheath and occupies the space inside the delivery sheath catheter. For another example, Chinese patent application No. CN201420301029.9 discloses a pressure-measurable medical catheter tip, in which a deformable body that deforms according to the change in force applied on it and a plurality of strain sensors provided on the deformable body are used to measure the force on the catheter tip. The catheter can only be measured for the force on the tip, but not on other parts of the catheter.

## SUMMARY

Accordingly, the present application provides a medical catheter that can adapt to larger-scale force or deformation measurement and has a compact structure, and a medical device including the medical catheter.

A medical catheter includes a catheter body and a resistance strain material layer. The resistance strain material layer is attached to a surface of the catheter body, or buried in the catheter body. The resistance strain material layer is deformable with bending and/or twisting of the catheter body.

Further, the resistance strain material layer includes a filament-shaped, foil-shaped, or film-shaped metal conductor made of a conductive material.

Further, the metal conductor adheres to the catheter body, or the metal conductor is attached to the catheter body by means of heat melting, spraying, or welding, or the metal conductor is provided on the catheter body by means of etching.

Further, the metal conductor is arranged to be curved in a square wave shape or a sine wave shape.

Further, the resistance strain material layer includes at least two metal conductors; wherein the metal conductors are parallel to each other, or the at least two metal conductors are symmetrically distributed on the catheter body with respect to at least one symmetry plane. An axis of the catheter body is located on the symmetry plane.

Further, the resistance strain material layer includes at least two metal conductors. Every two adjacent metal conductors are symmetrically arranged with respect to a symmetry plane passing through an axis of the catheter body.

Further, the metal conductors are arranged in a mesh shape.

Further, the resistance strain material layer includes at least two metal conductors. The at least two metal conductors are woven into a mesh structure which is provided on the catheter body to form the resistance strain material layer.

Further, the catheter body is a mesh tube structure woven from a first filament, and the resistance strain material layer is woven from a second filament. The second filament is co-woven into the mesh tube structure, wherein the first filament and the second filament are arranged to be insulated from each other, and the second filament is made of a conductive material.

Further, the first filament is made of an insulating material, or either the first filament or the second filament has a surface plated with an insulating film.

Further, a plurality of second filaments are provided and arranged parallel to each other.

Further, the resistance strain material layer includes a mesh unit formed by interweaving two second filaments. One of the second filaments constituting the mesh unit is helically co-woven into the mesh tube structure in a first direction, and the other of the second filaments constituting the mesh unit is helically co-woven into the mesh tube structure in a second direction that is opposite to the first direction.

Further, the resistance strain material layer includes at least two groups of the mesh units. The at least two groups of mesh units are arranged at intervals along a direction of an axial direction of the catheter body.

Further, the resistance strain material layer has at least a pair of test points.

Further, each of the test points is connected to a test position of the resistance strain material layer directly or via a wire.

Further, the test points are exposed on the surface of the catheter body, and/or the test points are arranged along a circumferential direction.

A medical catheter includes a catheter body and at least one resistance strain gauge combined with the catheter body. The resistance strain gauge is adapted to deform with stretching, bending, and/or twisting of the catheter body, wherein the at least one resistance strain gauge is attached to the surface of the catheter body, or buried in the catheter body, or embedded on a surface of the catheter body, or the at least one resistance strain gauge is co-woven into a woven material of the catheter body.

Further, the resistance strain gauge is a filament-shaped, foil-shaped or film-shaped resistance strain gauge made of a metal or semiconductor material.

Further, the resistance strain gauges are arranged in a serpentine, curved or helical shape on the catheter body; and/or, at least two resistance strain gauges intersect with each other on the catheter body.

Further, the medical catheter further includes at least a pair of test points electrically connected to the at least one resistance strain gauge.

A medical device includes the medical catheter as above.

The present application provides the medical device and the medical catheter thereof. The medical catheter includes the catheter body and the resistance strain material layer, wherein the resistance strain material layer is attached to the surface of the catheter body, or buried in the catheter body; and the resistance strain material layer is deformable with the bending and/or twisting of the catheter body, so that the strain of the resistive strain material layer would vary with the force on the medical catheter in actual operation. By measuring the resistance of the resistance strain material layer, the corresponding strain can be obtained, and thus the force on the catheter body can be obtained. In addition, since the resistance strain material layer is attached to the surface of the catheter body, or buried in the catheter body, and has a compact structure, which can adapt to the needs of providing a larger-scale resistance strain material layer on the catheter body to perform larger-scale force or deformation measurement.

## BRIEF DESCRIPTION OF THE DRAWINGS

To illustrate the technical solutions in the embodiments of the present application or in the prior art more clearly, the following briefly introduces the accompanying drawings required for describing the embodiments or the prior art. Apparently, the accompanying drawings in the following description show merely some embodiments of the present application, and persons of ordinary skill in the art may still derive other drawings from these accompanying drawings without creative efforts.

FIG. 1 is a schematic cross-sectional structure diagram of a medical catheter according to an embodiment.

FIG. 2 is a schematic cross-sectional structure diagram of a medical catheter according to another embodiment.

FIG. 3 is a schematic diagram of the structural morphology of a metal conductor forming a resistance strain material layer in a medical catheter according to an embodiment.

FIG. 4 is a schematic diagram of the structural morphology of metal conductors forming a resistance strain material layer in a medical catheter according to another embodiment, in which the arrangement of the metal conductors is shown.

FIG. 5 is a schematic diagram of the force on and strain of the plurality of metal conductors when the medical catheter shown in FIG. 4 is undergoing a bending test.

FIG. 6 is a schematic diagram of the structural morphology of metal conductors forming a resistance strain material layer in a medical catheter according to another embodiment.

FIG. 7 is a schematic distribution diagram of the structural morphology of metal conductors forming a resistance strain material layer in a medical catheter according to yet another embodiment.

FIG. 8 is a schematic structure diagram of a medical catheter according to another embodiment.

FIG. 9 is a schematic structure diagram of a medical catheter according to another embodiment.

FIG. 10 is a schematic structure diagram of a mesh unit formed by a second filament which forms a resistance strain material layer in a medical catheter according to yet another embodiment.

FIG. 11 is a schematic structure diagram of a plurality of mesh units forming a resistance strain material layer in a medical catheter according to another embodiment.

FIG. 12 is a schematic diagram of an implementation mode of testing a resistance strain material layer in a medical catheter according to an embodiment.

FIG. 13 is a schematic diagram of another implementation mode of testing a resistance strain material layer in a medical catheter according to an embodiment.

FIG. 14 is a schematic diagram of yet another implementation mode of testing a resistance strain material layer in a medical catheter according to an embodiment.

FIG. 15 is a schematic diagram of yet another implementation mode of testing a resistance strain material layer in a medical catheter according to an embodiment.

FIG. 16 is a schematic diagram of yet another implementation mode of testing a resistance strain material layer in a medical catheter according to an embodiment.

FIG. 17 is a schematic diagram of yet another implementation mode of testing a resistance strain material layer in a medical catheter according to an embodiment.

## DETAILED DESCRIPTION OF THE EMBODIMENTS

To make the present application easy to understand, a more comprehensive description of the present application will be given below with reference to the relevant drawings. Better embodiments of the present application are given in the drawings. However, the present application can be implemented in many different forms and shall not be limited to the embodiments described herein. The purpose of providing these embodiments is to provide a more thorough and comprehensive understanding of the application of the present application.

It is to be noted that when an element is referred to as being "fixed to" another element, it may be located on the other element directly or with intervening elements therebetween. When an element is referred to as being "connected to" another element, it may be connected to the other element directly or with intervening elements therebetween. The "connection" also includes a detachable connection. The terms "inner", "outer", "left", "right", and the like used herein are just for the purpose of description, rather than indicating a unique embodiment.

In an embodiment, a medical catheter is applicable in a medical device to form a transmission channel for an interventional medical device. The medical catheter includes a sheath catheter or other types of catheters. The interventional medical device may be, for example, a blood vessel stent or other medical devices that can be implanted into human's lumens such as a bile duct, esophagus, urethra, and the like. In some embodiments, the medical catheter can also be a push rod or other medical devices that need to have a measurement function.

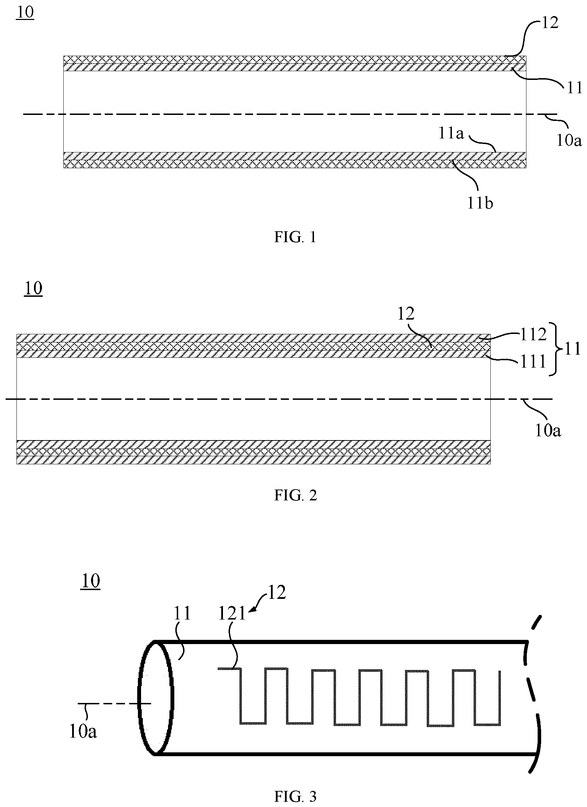

Referring to FIG. 1, a medical catheter 10 includes a catheter body 11 and a resistance strain material layer 12 provided on the catheter body 11. The resistance strain material layer 12 is combined with the catheter body 11. For example, in some embodiments, the resistive strain material layer 12 may be attached to a surface of the catheter body 11. Alternatively, in some embodiments, the resistance strain material layer 12 may be buried in the catheter body 11. It can be understood that the resistance strain material layer 12 can be combined with the catheter body 11 in other methods that can be conceived by those of ordinary skill in the art. The resistance strain material layer 12 can be deformed correspondingly with the stretching, bending, and/or twisting of the catheter body 11.

It should be noted that as a tubular structure, the catheter body 11 has a catheter wall that may be a solid catheter wall or a non-solid catheter wall.

In the case where the catheter wall of the catheter body 11 is a non-solid catheter wall, the catheter wall of the catheter body 11 may have a pierced structure, for example. At the pierced structure, the inner and outer surfaces of the catheter wall communicate with each other through the pierced structure. Alternatively, the catheter wall of the catheter body 11 can be a mesh structure, which is not limited herein. In the case where the catheter wall of the catheter body 11 is a solid catheter wall, the lumen of the catheter body 11 communicates with the outside, for example, through the opening at the end.

For the catheter body 11 with the solid catheter wall, the catheter body 11 has an inner surface 11a and an outer surface 11b disposed opposite to each other. The resistance strain material layer 12 can be attached to the inner surface 11a or the outer surface 11b. Also, the resistance strain material layer 12 may be embedded in the catheter body 11. For example, the resistance strain material layer 12 is attached to the outer surface 11b of the catheter body 11 in the medical catheter 10 shown in FIG. 1. For another example, the resistive strain material layer 12 is embedded in the catheter body 11 in the medical catheter 10 shown in FIG. 2. The embedding method of the resistance strain material layer 12 can be as follows. The catheter body 11 includes an inner layer structure 111 and an outer layer structure 112. When the medical catheter 10 is processed, a resistance strain material (for example, a resistance strain gauge) is sandwiched between the inner layer structure 111 and the outer layer structure 112, so that the resistance strain material layer 12 is embedded in the catheter body 11. In addition, the resistance strain material layer 12 can also be embedded in the catheter body 11 by integration with the catheter body 11. For example, when the catheter body 11 has a woven structure, the material forming the resistance strain material layer 12 can be woven into the woven structure of the catheter body 11 in a woven manner, which will be described in detail below, which is not detailed herein.

Taking the test of the tension of the medical catheter 10 as an example, the mechanical measurement principle of the medical catheter 10 will be described below.

Since the resistance strain material layer 12 can be deformed correspondingly with the deformation of the catheter body 11. For example, both of them are simultaneously subjected to stretching, compression, bending, twisting, etc., and form the medical catheter 10 with a certain tensile modulus. The corresponding tensile modulus can be obtained by performing a physical and mechanical test on the medical catheter 10. It can be understood that the tension of the medical catheter 10 when the medical catheter 10 is stretched is calculated as the product of the strain of the medical catheter 10 and the tensile modulus. The strain of the medical catheter 10 is consistent with the strain generated by the resistance strain material layer 12, so the electrical testing method can be used to obtain the change in the resistance value generated by the resistance strain material when being strained, so as to obtain the corresponding strain, and thus obtain the tension of the medical catheter 10.

In addition, the tension measurement of the medical catheter 10 can also be performed in the following method. Specifically, different tensions are directly applied to the medical catheter 10, and the resistance value of the resistance strain material layer 12 under the corresponding tension is respectively measured. From multiple sets of measurement data, a conversion formula between the resistance value and the tension of the medical catheter 10 is obtained. As such, when it is required to measure the tension of the medical catheter 10, only the resistance value of the resistance strain material layer 12 needs to be measured, and the tension of the medical catheter 10 can be obtained by the conversion formula.

Similarly, when it is required to measure the torque of the medical catheter 10, a corresponding conversion formula can also be obtained by applying different torques to the catheter and recording the resistance values of the torque strain gauge. Therefore, when monitoring the torque during the use of the medical catheter 10, the torque of the medical catheter 10 can be obtained by monitoring the resistance value.

It should be noted that the resistance value of the resistance strain material layer 12 is related to not only the properties of the material, but also the structure of the resistance strain material layer 12. Taking the resistance strain material layer 12 formed from the resistance strain gauge with a sheet-shaped structure as an example, the resistance value thereof is related to length, cross-sectional area, and other factors of the resistance strain gauge. As the resistance strain gauge deforms with the catheter body 11 being forced, the length and cross-sectional area of the resistance strain gauge, and thus its resistance, also change with the catheter body 11, following the formula as below:

$$dR/R = Ks*\varepsilon$$

where dR/R is the resistance change rate of the resistance strain gauge, Ks is the sensitivity coefficient of the resistance strain gauge material that physically means the resistance change rate per unit strain, indicating whether this type of resistance strain gauge has a significant effect, $\varepsilon$ is the strain at a measuring point, which is a dimensionless quantity, but is customarily still rendered with a unit named micro strain, usually represented by a symbol $\mu\varepsilon$. In addition, $\varepsilon$ can be measured with a resistance strainmeter.

It can be seen from the above that the strain $\varepsilon$ has a linear relationship with the resistance change rate dR/R when the resistance strain gauge produces a strain effect. That is, the strain $\varepsilon$ can be obtained from the resistance change rate.

In the medical catheter 10 of this embodiment, the resistance strain material layer 12 deforms along with the catheter body 11 when the medical catheter 10 is affected by an external force. The strain of the resistive strain material layer 12 would vary with the force on the medical catheter 10. By measuring the resistance of the resistance strain material layer 12, the corresponding strain, and thus the force on the catheter body 11, can be obtained. In addition, in the medical catheter 10 of this embodiment, the resistance strain material layer 12 is attached to the surface of the catheter body, or buried in the catheter body 11, and has a compact structure, which can adapt to the needs of providing a larger-scale resistance strain material layer 12 on the catheter body 11 to perform larger-scale force or deformation measurement. Therefore, the medical device employing the medical catheter 10 can be measured for the force on the medical catheter 10 in time when transporting medical equipment. Referring to the measured force, the operating force can be controlled appropriately, thereby avoiding damage to the blood vessel wall or tissue at the implantation site.

As shown in FIG. 3, in some embodiments, the resistance strain material layer 12 includes a filament-shaped, foil-shaped or film-shaped conductor 121 made of a conductive material. The conductor 121 may be made of a metal material, or a conductive semiconductor material. In some embodiments, the metal conductor 121 laminated on the surface of the catheter body 11 or sandwiched in the catheter body 11 forms the resistance strain material layer 12. In some embodiments, the conductor 121 is directly a resistance strain gauge. The metal conductor 121 may be, for example, adhered to the surface of the catheter body 11. Alternatively, the metal conductor 121 may be, for example, attached to the surface, or in the interlayer, of the catheter body 11 by means of thermal melting, spraying, welding, or the like. Alternatively, the metal conductor 121 may be arranged on the surface, or in the interlayer, of the catheter body 11 by means of chemical reaction etching.

The metal conductor 121 is arranged in a curved shape (such as a square wave shape or a square wave-like shape) or a serpentine shape or a helical shape. Such arrangement allows the metal conductors 121 to have better expansion and contraction performance, so as to better meet the needs of deformation with the catheter body 11, avoiding the limitation of the deformation of the medical catheter 10 due to the limited deformability of the metal conductors 121, or avoiding the influence on stress measurement by the poor deformability and easy damage of the metal conductor 121. In other embodiments, the metal conductors 121 may also be arranged in a sine wave shape.

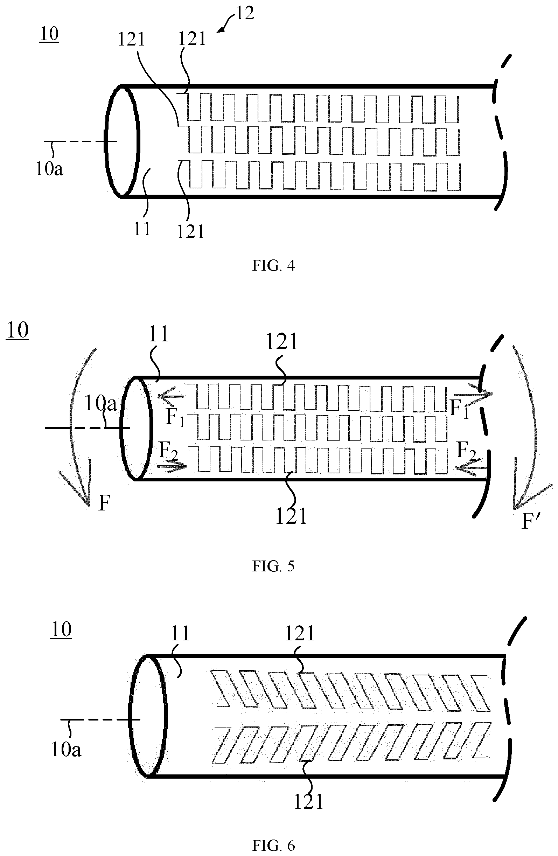

As shown in FIG. 4, in some embodiments, the resistance strain material layer 12 includes a plurality of metal conductors 121 which are parallel to each other. Thus, when the medical catheter 10 is bent, the resistances measured by the plurality of metal conductors 121 can be used to obtain the tension at different positions. From the tension difference between opposite sides in the bending direction, the bending moment and the bending angle of the medical catheter 10 can be obtained.

As shown in FIG. 5, when the medical catheter 10 is subjected to a bending force F-F', the upper metal conductor 121 is subjected to a pulling force F1 that stretches outward from the middle to both ends, and the lower metal conductor 121 is subjected to a pressure F2 that is squeezed inward from both ends. From the tension difference formed between the pulling force F1 and the pressure F2, the bending degree or the bending moment of the current medical catheter 10 is obtained.

As shown in FIG. 6, in some embodiments, the plurality of (two or more) metal conductors 121 are symmetrically distributed on the catheter body 11 with respect to at least one symmetry plane, wherein an axis 10a of the catheter body 10 is located on the symmetry plane. With this symmetrical arrangement, the pattern formed by the resistance strain material layer 12 can be made more regular, and the deformability is equivalent on various parts of the medical catheter 10. As such, when deformation occurs, there is a small difference in strain caused by the same stress at different parts of the medical catheter 10, so that it is convenient to unify the force measurement standards on various parts of the medical catheter 10. There is no need for special conversion standards due to the different stresses of the local resistance strain material layer 12, thereby reducing the difficulty of force measurement of the medical catheter 10.

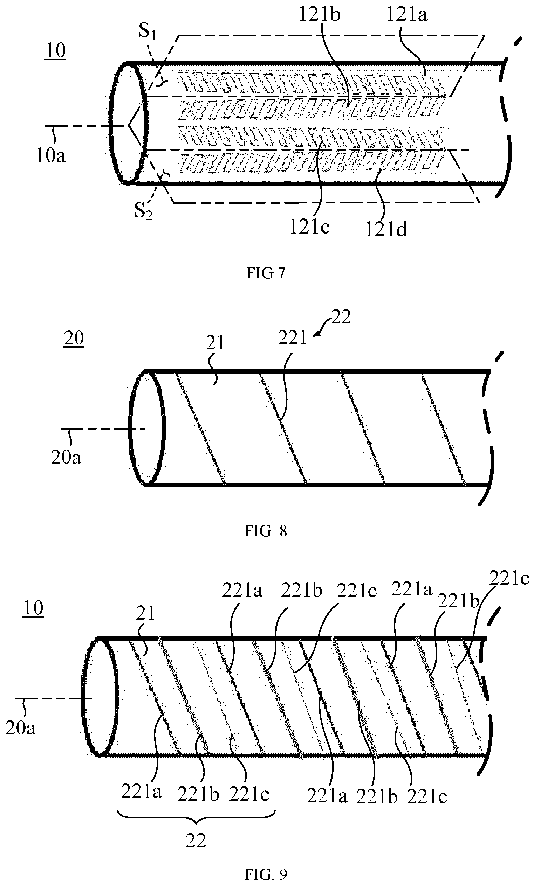

As shown in FIG. 7, in some embodiments, the resistance strain material layer 12 includes a plurality of metal conductors 121. Adjacent metal conductors 121 are symmetrically arranged with respect to a symmetry plane that is a geometric plane passing through the axis 10a of the catheter body 11. For ease of description, in the medical catheter 10 shown in FIG. 7, two different planes passing through the axis 10a of the catheter body 11 are established as virtual symmetry planes, i.e. a first symmetry plane S1 and a second symmetry plane S2, respectively. The metal conductor 121a and the metal conductor 121b are symmetrically arranged with respect to the first symmetry plane S1, and the metal conductor 121c and the metal conductor 121d are symmetrically arranged with respect to the second symmetry plane S2. It can be understood that the metal conductor 121b and the metal conductor 121c may also be symmetrically arranged with respect to one symmetry plane.

The metal conductor 121 may also have other structural forms. For example, in some embodiments, the metal conductor 121 is arranged in a mesh shape. Specifically, a plurality of metal conductors 121 are provided and woven into a mesh structure. The mesh structure is provided on the catheter body 11 to form the resistance strain material layer 12. For example, the plurality of metal conductors 121 may be woven into the mesh structure first, and then the mesh structure is covered on the catheter body 11 to form the medical catheter 10. Alternatively, the plurality of metal conductors 121 may be arranged in the catheter body 11 in a manner of covering or being buried or embedding to form the mesh structure in the process of processing the medical catheter 10.

In some embodiments, the catheter body 11 may be formed by extrusion first, and then a single or a plurality of metal conductors are wound on the surface of the catheter body and melted to form the resistance strain material layer 12. In other embodiments, a heat-shrinkable tube may be wrapped on the outer layer of the resistance strain material layer 12. The resistance strain material layer 12 is fixed to the catheter body 11 using the heat-shrinkable tube.

As shown in FIG. 8, the present application provides another embodiment of a medical catheter 20. The medical catheter 20 includes a catheter body 21 and a resistance strain material layer 22. The resistance strain material layer 22 is combined with the catheter body 21, and the resistance strain material layer 22 can be deformed correspondingly with the stretching, bending, and/or twisting of the catheter body 11. Compared with the above-mentioned embodiments, the difference is that the catheter body 21 is a mesh tube structure woven from a first filament (not shown in the figure). The resistance strain material layer 22 is woven from a second filament 221. The second filament 221 is co-woven into the mesh tube structure. The first filament and the second filament 221 are arranged to be insulated from each other. The second filament 221 is made of a conductive material. The second filaments 221 may be co-woven into the mesh tube structure in a helical shape, or may be co-woven into the mesh tube structure in a straight line, a zigzag shape, or other manners.

In some embodiments, the first filament is made of an insulating material, so that there is no current between the catheter body 21, which is formed by weaving the first filament, and the second filament 221, so that when the resistance strain material layer 22 formed by the second filament 221 is tested, the resistance strain material layer 22 can accurately reflect the strain of the resistance strain material layer 22, so as to improve the measurement accuracy of the force on the medical catheter 20.

In other embodiments, a surface of the first filament or the second filament 221 is plated with an insulating film. As such, even if the first filament, or the catheter body 21, is made of a conductive material, the resistance strain measurement of the second filament 221 can be achieved using the insulating film without interference, so as to improve the measurement accuracy of the force on the medical catheter 20.

In some embodiments, a plurality of (two or more) second filaments 221 are provided, and are arranged in parallel.

To clearly present the parallel arrangement of the plurality of second filaments 221 on the catheter body 21, as shown in FIG. 9, FIG. 9 schematically shows three second filaments with different wire diameters, which are specifically a second filament 221a, a second filament 221b, and a second filament 221c. It should be noted that the different diameters of the second filaments 221a, 221b, and 221c as shown are only for the purpose of clearly presenting the structure of parallel arrangement of the three second filaments. In fact, the plurality of second filaments 221 may have the same or different diameters, which is not limited herein.

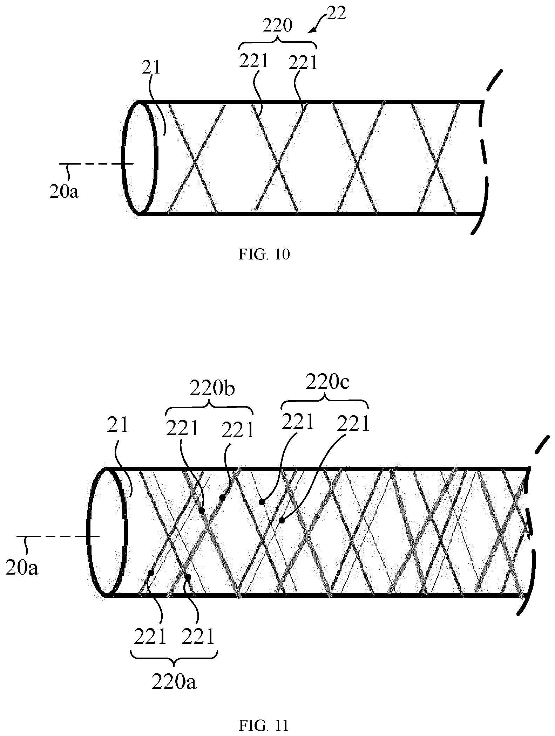

As shown in FIG. 10, in some embodiments, the resistance strain material layer 22 includes a mesh unit 220 formed by interweaving two second filaments 221, wherein one of the second filaments 221 constituting the mesh unit 220 is helically co-woven into the mesh tube structure in a first direction, and the other of the second filaments 221 constituting the mesh unit 220 is helically co-woven into the mesh tube structure in a second direction that is opposite to the first direction. Specifically, the two second filaments 221 constituting the mesh unit 220 are arranged on the catheter body 21 helically around an axis 20a of the catheter body 21 in a positive helix and a negative helix, respectively.

With the configuration of this structure, such a helical structure is more susceptible to the torsion moment to produce strain when the medical catheter 20 is tested for torque, which results in that it is more conducive to obtain the torsion moment of the medical catheter 20 by measuring the resistance value of the resistance strain material layer 22.

Further, a plurality of mesh units 220 are provided and arranged at intervals along the direction of the axis 20a of the catheter body 21. The provision of the plurality of mesh units 220 is for increasing the density of measurement, so that higher measurement accuracy can be obtained when the force measurement is performed on various parts of the medical catheter 20.

As shown in FIG. 11, in some embodiments, taking three mesh units of a mesh unit 220a, a mesh unit 220b, and a mesh unit 220c as an example, each mesh unit includes two second filaments 221 that are interwoven with each other. Meanwhile, there are gaps between the mesh unit 220a, the mesh unit 220b, and the mesh unit 220c along the direction of the axis 20a of the duct body 21, that is, they are arranged at intervals along the axial direction. To be precise, the second filaments 221 constituting the plurality of mesh units 220 do not overlap with each other. It is understandable that two, or three or more mesh units 220 may be provided, which is not limited herein.

In some embodiments, the resistance strain material layer 12 has several test points, and the test points are exposed on the surface of the catheter body 11. The resistance strain material layer 12 is measured through the test points, so as to obtain the force on the medical catheter 10.

In the following, the method of measuring the resistance value of the resistance strain material with an ohmmeter as a measuring instrument is described.

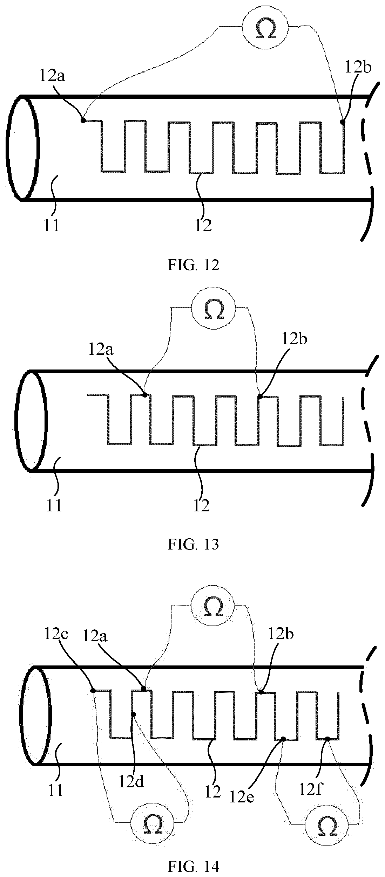

As shown in FIG. 12, the ohmmeter is connected to a test point 12a and a test point 12b located at the two ends of the resistance strain material layer 12, and the entire section of the resistance strain material layer 12 is measured.

As shown in FIG. 13, the test point 12a and the test point 12b are located in the middle part of the resistance strain material layer 12, so that the resistance value of the part between the test point 12a and the test point 12b can be measured by the ohmmeter when the ohmmeter is connected.

In addition, multiple ohmmeters can be used to measure the resistance of multiple sections by setting multiple test points.

For example, as shown in FIG. 14, the resistance strain material layer 12 is provided with a test point 12a, a test point 12b, a test point 12c, a test point 12d, a test point 12e, and a test point 12f. The resistance value of the part between the test point 12a and test point 12b can be measured by the ohmmeter connected between the test point 12a and test point 12b. Similarly, the resistance value of the part between the test point 12c and test point 12d can be measured by the ohmmeter connected between the test point 12c and test point 12d. The resistance value of the part between the test point 12e and test point 12f can be measured by the ohmmeter connected between the test point 12e and test point 12f. Through this method of segmented measurement, the corresponding force on each segment of the catheter 10 can be obtained and output respectively.

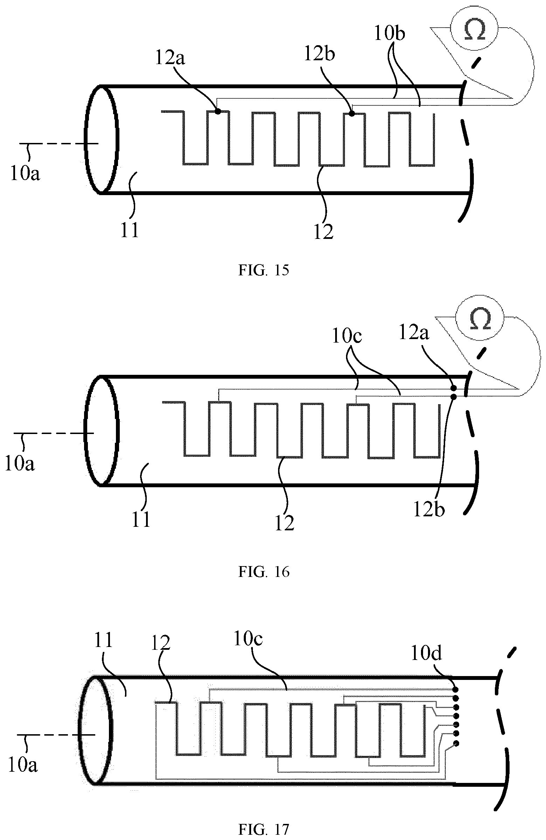

As shown in FIG. 15, when measuring the resistance value, the wires connected to the ohmmeter can be arranged in a direction substantially parallel to the axis of the catheter body 11 and be connected to the corresponding test points 12a and 12b.

As shown in FIG. 16, a wire connected to the resistive strain material layer 12 can be provided on the catheter body 11 in advance. The wire leads to the corresponding test point 12a and test point 12b, so that the positions of the test point 12a and the test point 12b can be arranged in suitable positions according to actual needs, so as to facilitate the connection with the ohmmeter during measurement. For example, the test point 12a and test point 12b may be arranged along the circumferential direction of the catheter body 11.

It should be noted that two or more test points can be provided.

For example, as shown in FIG. 17, a plurality of test points 10d are arranged along the circumference direction of the catheter body 11. The catheter body 11 is provided with wires 10c that connect the test points 10d with different test positions of the resistive strain material layer 12. With this arrangement, the test points 10d are concentrated in a relatively regular manner, and the wires 10c are arranged more orderly on the catheter body 11, so that the wires 10c will not interfere with other structures when the force on the medical catheter 10 is measured. Only the connection between the ohmmeter and other instruments to the corresponding test points 10d is required for measurement.

The medical catheter 10 provided in the present application can be used to detect the degree of calcification of tissues such as blood vessels, and can also be used in a device for measuring ligation force when ligating tissues (such as myocardium) in the interventional surgery, so as to accurately control the ligation force, which can reduce adverse reactions caused by tight or loose ligation. In addition to the application scenarios as described above, the medical catheter 10 can also be used in other scenarios where the tension, stress, strain, torque, or bending moment in vivo needs to be measured.

In some embodiments, the medical catheter includes the catheter body 11 and at least one resistance strain gauge combined with the catheter body 11. The resistance strain gauge is adapted to deform with the stretching, bending, and/or twisting of the catheter body 11. The deformation of the resistance strain gauge (for example, the change in length, the change in cross-sectional area) brings about the change in its own resistance value, so that the force on the catheter body 11 can be inferred. Similar to the combination of the metal conductor and the catheter body 11 in the above embodiments, the resistance strain gauge can be attached to the surface of the catheter body 11, or be buried in the catheter body 11, or embedded on the surface of the catheter body 11. Alternatively, when the catheter body 11 is formed by weaving, the resistance strain gauge can be directly co-woven into the woven material of the catheter body 11.

In some embodiment, the resistance strain gauge is a filament-shaped, foil-shaped or film-shaped resistance strain gauge made of a metal or semiconductor material.

In some embodiments, the resistance strain gauges are arranged in a serpentine, curved, or helical shape on the catheter body 11; and/or, at least two resistance strain gauges intersect with each other on the catheter body 11.

In some embodiments, the medical catheter further includes at least a pair of test points electrically connected to the at least one resistance strain gauge. The at least a pair of test points can be provided with reference to the test points in the foregoing embodiments.

In some embodiments, the length of the catheter body 11 on which the resistance strain gauges are arranged occupies more than half, or more than two-thirds, or more than three-quarters, of the entire length of the catheter body.

The technical features of the embodiments described above may be arbitrarily combined. For the sake of brevity of description, not all possible combinations of the technical features in the aforementioned embodiments are described. However, as long as there is no contradiction between the combinations of these technical features, all should be considered to be fallen within the scope of this specification.

The above embodiments only represent several examples of the present application, and the description thereof is more specific and detailed, but it should not be construed as limiting the scope of the present application. It should be noted that for those of ordinary skill in the art, without departing from the concept of the present application, several modifications and improvements can be made, and these all fall within the protection scope of the present application. Therefore, the protection scope of the patent in the present application shall be subject to the appended claims.

**Claims**

1. A medical catheter, comprising a catheter body and a resistance strain material layer, wherein the resistance strain material layer is attached to a surface of the catheter body, or buried in the catheter body; and the resistance strain material layer is deformable with bending and/or twisting of the catheter body.

2. The medical catheter according to claim 1, wherein the resistance strain material layer comprises a filament-shaped, foil-shaped, or film-shaped metal conductor made of a conductive material.

3. The medical catheter according to claim 2, wherein the metal conductor adheres to the catheter body, or the metal conductor is attached to the catheter body by means of heat melting, spraying, or welding, or the metal conductor is provided on the catheter body by means of etching.

4. The medical catheter according to claim 2 or 3, wherein the metal conductor is arranged to be curved in a square wave shape or a sine wave shape.

5. The medical catheter according to claim 3 or 4, wherein

   the resistance strain material layer comprises at least two metal conductors;
   wherein the metal conductors are parallel to each other, or the at least two metal conductors are symmetrically distributed on the catheter body with respect to at least one symmetry plane, and an axis of the catheter body is located on the symmetry plane.

6. The medical catheter according to claim 3 or 4, wherein the resistance strain material layer comprises at least two metal conductors, and every two adjacent metal conductors are symmetrically arranged with respect to a symmetry plane passing through an axis of the catheter body.

7. The medical catheter according to claim 2 or 3, wherein the metal conductors are arranged in a mesh shape.

8. The medical catheter according to claim 2 or 3, wherein the resistance strain material layer comprises at least two metal conductors, the at least two metal conductors being woven into a mesh structure which is provided on the catheter body to form the resistance strain material layer.

9. The medical catheter according to claim 1, wherein the catheter body is a mesh tube structure woven from a first filament, and the resistance strain material layer is woven from a second filament, the second filament is co-woven into the mesh tube structure, wherein the first filament and the second filament are arranged to be insulated from each other, and the second filament is made of a conductive material.

10. The medical catheter according to claim 9, wherein the first filament is made of an insulating material, or either the first filament or the second filament has a surface plated with an insulating film.

11. The medical catheter according to claim 9, wherein a plurality of second filaments are provided and arranged parallel to each other.

12. The medical catheter according to claim 9, wherein the resistance strain material layer comprises a mesh unit formed by interweaving two second filaments, wherein one of the second filaments constituting the mesh unit is helically co-woven into the mesh tube structure in a first direction, and the other of the second filaments constituting the mesh unit is helically co-woven into the mesh tube structure in a second direction that is opposite to the first direction.

13. The medical catheter according to claim 12, wherein the resistance strain material layer comprises at least two groups of the mesh units, the at least two groups of mesh units being arranged at intervals along a direction of an axial of the catheter body.

14. The medical catheter according to any one of claims 1-13, wherein the resistance strain material layer has at least a pair of test points.

15. The medical catheter according to claim 14, wherein each of the test points is connected to a test position of the resistance strain material layer directly or via a wire.

16. The medical catheter according to claim 15, wherein the test points are exposed on the surface of the catheter body, and/or the test points are arranged along a circumferential direction.

17. A medical catheter, comprising a catheter body and at least one resistance strain gauge combined with the catheter body, the resistance strain gauge being adapted to deform with stretching, bending, and/or twisting of the catheter body, wherein the at least one resistance strain gauge is attached to a surface of the catheter body, or buried in the catheter body, or embedded on a surface of the catheter body, or the at least one resistance strain gauge is co-woven into a woven material of the catheter body.

18. The medical catheter according to claim 17, wherein the resistance strain gauge is a filament-shaped, foil-shaped, or film-shaped resistance strain gauge made of a metal or semiconductor material.

19. The medical catheter according to claim 17 or 18, wherein the resistance strain gauges are arranged in a serpentine, curved, or helical shape on the catheter body; and/or, at least two resistance strain gauges intersect with each other on the catheter body.

20. The medical catheter according to any one of claims 17-19, wherein the medical catheter further comprises at least a pair of test points electrically connected to the at least one resistance strain gauge.

21. A medical device, comprising the medical catheter according to any one of claims 1-20.

<u>10</u>

12

11

11a

10a

11b

FIG. 1

<u>10</u>

12

112
111 } 11

10a

FIG. 2

<u>10</u>

11  121  12

10a

FIG. 3

FIG. 4

FIG. 5

FIG. 6

10

S₁

121b    121a

10a

S₂

121c    121d

FIG.7

20

21    221    22

20a

FIG. 8

10

21    221a  221b  221c  221a  221b  221c

20a

221a  221b  221c    221a  221b  221c

22

FIG. 9

FIG. 10

FIG. 11

FIG. 12

FIG. 13

FIG. 14

FIG. 15

FIG. 16

FIG. 17

## INTERNATIONAL SEARCH REPORT

International application No.

**PCT/CN2020/091573**

| A. | CLASSIFICATION OF SUBJECT MATTER |
|---|---|

G01L 1/20(2006.01)i; A61M 25/00(2006.01)i

According to International Patent Classification (IPC) or to both national classification and IPC

| B. | FIELDS SEARCHED |
|---|---|

Minimum documentation searched (classification system followed by classification symbols)

G01L; A61M; A61B

Documentation searched other than minimum documentation to the extent that such documents are included in the fields searched

Electronic data base consulted during the international search (name of data base and, where practicable, search terms used)

VEN; CNABS; CNTXT: test point?, 应变, 应变计, 应变仪, 电阻应变, 应变材料, 多根, 扭转, 管壁, 壁, 弯曲, 埋, 扭曲, 扭矩, 大面积, 大幅面, 喷涂, 热熔, 焊接, 螺旋, 电阻, 阻值, 对称, 矩形, 正弦, 电阻丝, 弯矩, 贴附, 形变, 导管, 鞘管, 管, 网, 编织, pipe, tube, sheath, stress, bend, resistance, strain, wire, gague, weave, catheter

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 109141697 A (SHANGHAI MICROPORT EP MEDTECH CO., LTD.) 04 January 2019 (2019-01-04)<br>  paragraphs 63-116, and figures 1-16 | 17-21 |
| X | CN 105434035 A (SHANGHAI MICROPORT EP MEDTECH CO., LTD.) 30 March 2016 (2016-03-30)<br>  paragraphs 55-62, and figures 1-3 | 17-21 |
| X | CN 203915066 U (LEPU MEDICAL TECHNOLOGY (BEIJING) CO., LTD.) 05 November 2014 (2014-11-05)<br>  paragraphs 34-37, and figures 1-6 | 17-21 |
| A | CN 105050491 A (GRAFTWORX, LLC) 11 November 2015 (2015-11-11)<br>  entire document and figure | 1-21 |
| A | CN 204581494 U (ZHENGZHOU FUJIANDA MEDICAL EQUIPMENT CO., LTD.) 26 August 2015 (2015-08-26)<br>  entire document and figure | 1-21 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 August 2020** | **26 August 2020** |

| Name and mailing address of the ISA/CN | Authorized officer |
|---|---|
| **China National Intellectual Property Administration (ISA/ CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

| International application No. |
| --- |
| **PCT/CN2020/091573** |

| Patent document cited in search report | | | Publication date (day/month/year) | Patent family member(s) | | | Publication date (day/month/year) |
| --- | --- | --- | --- | --- | --- | --- | --- |
| CN | 109141697 | A | 04 January 2019 | EP | 3640578 | A4 | 17 June 2020 |
| | | | | WO | 2018228290 | A1 | 20 December 2018 |
| | | | | CN | 109141697 | B | 10 April 2020 |
| | | | | EP | 3640578 | A1 | 22 April 2020 |
| | | | | US | 2020100859 | A1 | 02 April 2020 |
| CN | 105434035 | A | 30 March 2016 | CN | 105434035 | B | 20 March 2018 |
| CN | 203915066 | U | 05 November 2014 | | None | | |
| CN | 105050491 | A | 11 November 2015 | US | 2020155063 | A1 | 21 May 2020 |
| | | | | EP | 2948049 | A4 | 19 October 2016 |
| | | | | JP | 6356152 | B2 | 11 July 2018 |
| | | | | US | 9427305 | B2 | 30 August 2016 |
| | | | | AU | 2014209143 | A1 | 06 August 2015 |
| | | | | CN | 105050491 | B | 11 July 2017 |
| | | | | WO | 2014117037 | A1 | 31 July 2014 |
| | | | | US | 2014214149 | A1 | 31 July 2014 |
| | | | | EP | 2948049 | A1 | 02 December 2015 |
| | | | | US | 10548527 | B2 | 04 February 2020 |
| | | | | US | 10542931 | B2 | 28 January 2020 |
| | | | | US | 2016331312 | A1 | 17 November 2016 |
| | | | | CA | 2898857 | A1 | 31 July 2014 |
| | | | | US | 2016331313 | A1 | 17 November 2016 |
| | | | | JP | 2016511656 | A | 21 April 2016 |
| | | | | AU | 2014209143 | B2 | 24 May 2018 |
| CN | 204581494 | U | 26 August 2015 | | None | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201520229910

- CN 201420301029